# EUROPEAN PATENT SPECIFICATION

(11) **EP 0 566 371 B1**
(45) Date of publication and mention of the grant of the patent: **29.01.1997**
(21) Application number: 93302873.0
(22) Date of filing: 14.04.1993
(51) Int. Cl.: C07C 69/16, C07C 67/37, C07C 51/54, C07C 53/12, C07C 69/14

(54) **Process for converting dimethyl ether to ethylidene diacetate**
Verfahren zur Umwandlung von Dimethyläther in Äthylidendiacetat
Procédé de conversion de l'éther diméthylique en diacétate d'éthylidène

(30) Priority: 15.04.1992 US 870126
(43) Date of publication of application: 20.10.1993
(73) Proprietor: AIR PRODUCTS AND CHEMICALS, INC., Allentown, PA 18195-1501 (US)
(72) Inventor: Waller, Francis Joseph, Allentown, PA 18103-9670 (US)
(74) Representative: Burford, Anthony Frederick

(56) References cited:
- EP-A- 0 077 116
- GB-A- 1 538 782

## Description

This invention relates to a process for producing ethylidene diacetate by reacting dimethyl ether, acetic acid, hydrogen and carbon monoxide in the presence of a novel catalytic system comprising a Group VIII metal, methyl iodide, lithium iodide and, optionally, lithium acetate.

Ethylidene diacetate (EDDA) and acetic acid are valuable chemicals which can be used as solvents or as intermediates in preparing a wide variety of commercially valuable compositions such as vinyl acetate, methyl acetate and acetic anhydride. Considerable interest has been focused on developing improved processes for preparing ethylidene diacetate wherein problems associated with prior art methods can be overcome.

Representative processes for preparing EDDA include US-A-4,429,150 which discloses a process for producing EDDA wherein methyl acetate and/or dimethyl ether, carbon monoxide and hydrogen are reacted in the presence of a catalyst system comprising a Group VIII metal and a halogen-containing compound in the presence of a sulphur-containing polar solvent, e.g. sulpholane. The reference teaches that organo-phosphorus compounds improve selectivity and increase conversion to EDDA.

US-A-4,323,697 discloses a process for preparing EDDA wherein methyl acetate and/or dimethyl ether, carbon monoxide and hydrogen are reacted in the presence of a molybdenum-nickel or tungsten-nickel co-catalyst in the presence of a promoter comprising an organo-phosphorus compound or an organo-nitrogen compound. When dimethyl ether is utilized, the reference teaches that a reactor having two reaction zones is preferred. In the first zone, DME is converted by carbonylation to methyl acetate and the second zone is devoted to conducting the EDDA forming-reaction.

GB-A-1,538,782 discloses a process for producing EDDA wherein dimethyl ether and/or methyl acetate, carbon monoxide and hydrogen are reacted in the presence of a catalyst system. Example 17 teaches that methyl acetate and dimethyl ether can be reacted in the vapor phase in the presence of a source of halide, e.g. methyl bromide or iodide, acetyl bromide or iodide, hydrogen bromide or iodide or mixtures thereof. The reaction preferably occurs in the presence of a Group VIII metal catalyst and a promoter such as an organo-phosphine and/or organo-nitrogen compound.

Considerable interest abounds in discovering a one-step process for preparing EDDA in high yield under substantially anhydrous conditions. The instant patent application presents a process which overcomes many of the limitations associated with prior art processes. Specifically, the instant process provides high selectivity to EDDA under shorter reaction times than disclosed in prior art processes.

The present invention relates to an improved process for preparing ethylidene diacetate (EDDA) which utilizes a newly discovered catalyst system which provides superior selectivity to EDDA and which can be operated under shorter reaction times than typically required in prior art processes for producing EDDA. The newly discovered catalyst system consists essentially of a Group VIII metal, methyl iodide, lithium iodide and optionally, lithium acetate, which in combination, provide superior results than achieved in prior art processes for producing EDDA which utilize a catalyst system containing less than the combination of these components.

The instant process for producing EDDA comprises reacting dimethyl ether (DME), hydrogen, carbon monoxide and acetic acid in the presence of a catalyst system consisting essentially of a Group VIII metal, methyl iodide, lithium iodide and optionally, lithium acetate under conditions sufficient to form a reaction mixture comprising EDDA and acetic acid. The desired product, EDDA, is recovered from the product mixture which includes acetic acid and other reaction products. As will be evident from this Specification, the present invention provides a convenient route for coproducing EDDA and acetic acid.

The term, hydrocarbonylation, as referred to herein, refers to the reaction of dimethyl ether, acetic acid, hydrogen and carbon monoxide to form EDDA under the enumerated process conditions. Hydrocarbonylation can be carried out in a batch mode or a continuous mode over a wide range of temperatures. While the optimum temperature for practicing the present invention will depend upon process stoichiometry, the particular catalyst system utilized, as well as the precise combination of reaction conditions, suitable hydrocarbonylation temperatures will range from 20°C up to 220°C. However, the most preferred hydrocarbonylation temperatures range from 120°C to 195°C. The hydrocarbonylation reaction can be carried out under a wide variety of pressures including pressures ranging from 100 psig (0.8 MPa) to 3000 psig (21 MPa). Preferred pressures range from 400 psig (2.75 MPa) to 2100 psig (14.5 MPa).

The catalyst system of the present invention utilizes a Group VIII metal selected from the group consisting of rhodium, platinum, palladium, iridium, ruthenium, cobalt and nickel with preferred Group VIII metals being rhodium and iridium. The Group VIII metal catalyst used in the catalyst system is present in a catalytically active amount and such catalytically effective amounts can be readily determined by those of ordinary skill in the art. The amount of Group VIII metal to be incorporated into the catalyst system typically ranges from 0.01 mol% to 10 mol% based on the DME present, preferably from 0.05 to 5 mol%. The most preferred Group VIII metal is rhodium.

Examples of suitable rhodium compounds to be incorporated into the catalyst system include rhodium oxide, rhodium (III) hydroxide, rhodium (III) chloride, rhodium (III) chloride trihydrate, rhodium (III) bromide, rhodium (III) iodide, rhodium (II) acetate, tetrarhodium dodecacarbonyl, hexarhodium hexadecacarbonyl, rhodium (I) dicarbonyl acetylacetonate, tris(pyridine)rhodium (III) chloride, chlorotris-(triphenylphosphine) rhodium and other organo-rhodium complexes. The preferred rhodium compound to be incorporated into the catalyst system is rhodium (III) chloride trihydrate which is commercially available in hydrated forms.

Examples of suitable palladium compounds to be incorporated into the catalyst system include palladium chloride, palladium chloride dihydrate, palladium bromide, palladium iodide, palladium oxide, palladium acetate, palladium butyrate and palladium acetylacetonate. Preferred palladium compounds include palladium chloride, palladium chloride dihydrate and palladium acetate.

In addition to the Group VIII metal, the catalyst system also contains methyl iodide and lithium iodide, in combination, which serve as promoters for driving the hydrocarbonylation reaction to completion. Applicant has discovered that unexpectedly superior conversion of dimethyl ether to EDDA occurs when lithium acetate is used in conjunction with both lithium iodide and methyl iodide. The amount of lithium iodide and methyl iodide used in conjunction with the desired Group VIII metal catalyst is not critical to practicing the present invention. The collective amount of the iodide components, (i.e., methyl iodide and lithium iodide) can be varied between wide limits.

Reaction time is not critical in practicing the present invention and one of ordinary skill in the art can determine optimum reaction times based upon the enumerated reaction conditions, catalyst system and catalyst concentration presented herein. Reaction times required to produce a desired amount of EDDA will also depend upon the reaction temperature and pressure. The reaction is preferably run in acetic acid which is a reactant to the present process as well as a coproduct produced therefrom. A dipolar solvent may be utilized in conjunction with acetic acid.

The following examples are presented to further illustrate the scope of the present invention and are not intended to limit the scope thereof.

### EXAMPLES

### EXPERIMENTAL PROCEDURE

The reaction products were analyzed by gas chromatography using a DB-1701 FSOT capillary column interfaced to a flame ionization detector. Quantitation was obtained using an internal standard technique. The lower detection limit for the compounds of interest was approximately 0.002 wt.%. All organic compound structures were verified by gas chromatography/mass spectrometry (GC/MS).

A 300 cm³ Hastelloy C autoclave was equipped with a dip tube for loading DME from a preweighed cylinder, thermocouple, cooling coils, a belt driven magnetic stirrer and an inlet for gases. The autoclave was protected from overpressure by a rupture disk and a relief valve. All inlet lines, valves and other surfaces being exposed to methyl iodide were made of either Hastelloy C or Inconel.

The following general procedure was used to load, pressurize, run and unload the autoclave. The autoclave was charged with acetic acid, Group 1a salt(s), methyl iodide and other components mentioned in the Tables below. The autoclave was sealed, pressurized with nitrogen to test for leaks, vented, pressurized with a synthesis gas (syngas) pre-mix at least thrice, and vented to approximately 20 psig (140 MPa). DME was transferred to the autoclave from a preweighed cylinder. While stirring, the syn-gas pressure was increased to 300-400 psig (2-2.75 MPa) and the reactor was heated to operating temperature. At operating temperature, reactor pressure was increased to operating pressure. The reactions were run for the desired length of time while the autoclave was maintained at constant pressure. Following completion of the reaction, the autoclave was cooled to room temperature, depressurized and the contents were decanted from the reactor. The reactor was rinsed with 25 ml acetic acid and combined with the reactor discharge.

### EXAMPLES 1-6

Examples 1 - 6 set forth in Table 1 demonstrate that EDDA can be produced by hydrocarbonylating DME using a CO/H₂ mix of 1:1 and the enumerated catalyst systems in acetic acid. In the selectivity calculation, Σ(mmol products) was calculated according to the sum of (MeOAc + Ac₂O + AcH + EDDA) and is based upon the DME required to form each product. According to Examples 1, 2 and 6 of Table 1 and Example 7 of Table 2, an upside down volcano relationship was found to exist between EDDA selectivity and mol % LiI/(mol LiI + mol MeI) used in the catalyst system wherein the ratio of DME to MeI was held constant. An inverse relationship was observed in Example 6 of Table 1 and Examples 15 and 16 in Table 4 wherein the ratio of DME to LiI was held constant.

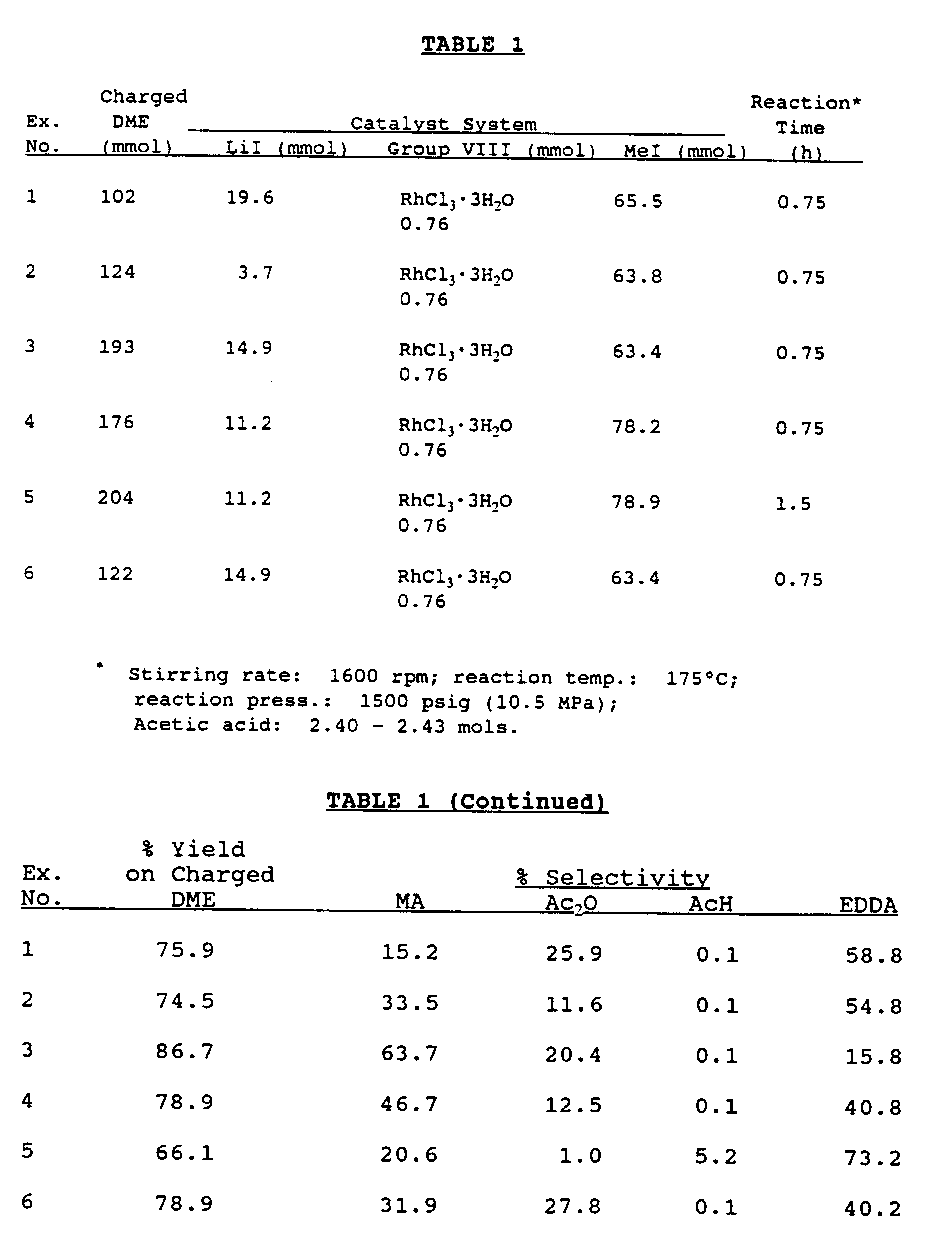

### EXAMPLES 7-10

Examples 7 - 10 set forth in Table 2 use a CO/H₂ mix of 1:1 and demonstrate results obtained by reacting DME, acetic acid, hydrogen and carbon monoxide in the presence of the enumerated catalyst systems. The number of mmol of EDDA as defined in Table 2 was the actual amount of DME required to prepare the GC measured amount of EDDA. The reported amount of acetic acid in Table 2 is the incremental amount above the charged acetic acid and corresponds to the amount expected when EDDA and acetic acid are coproduced.

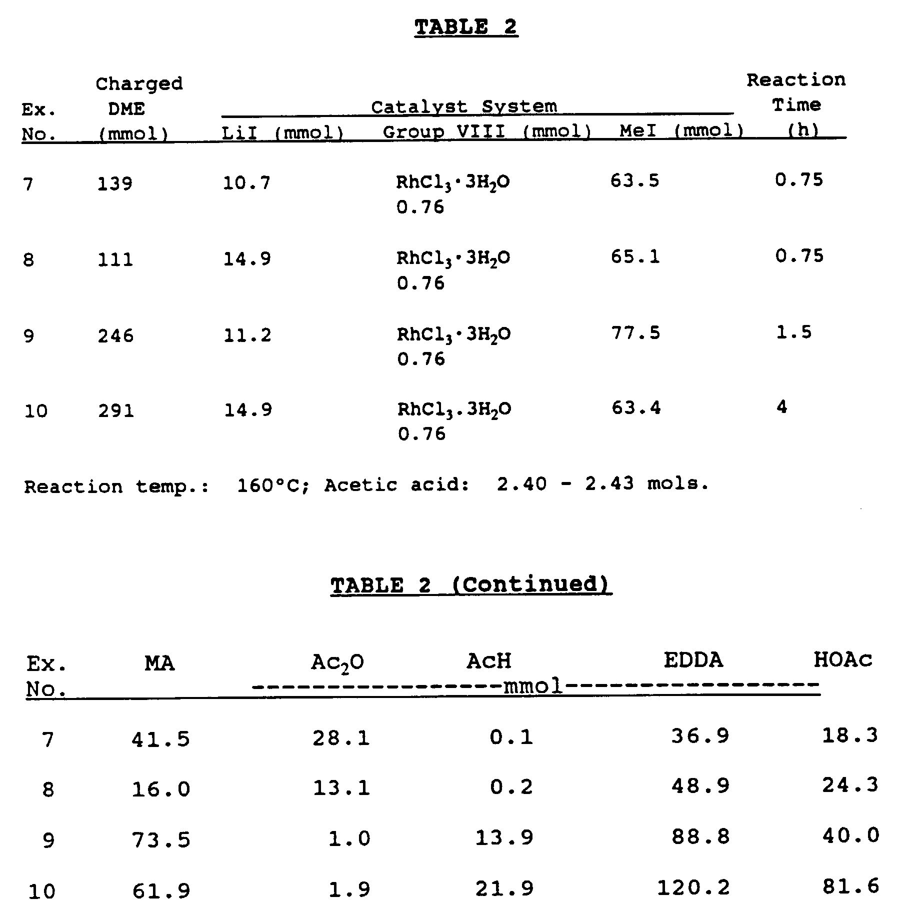

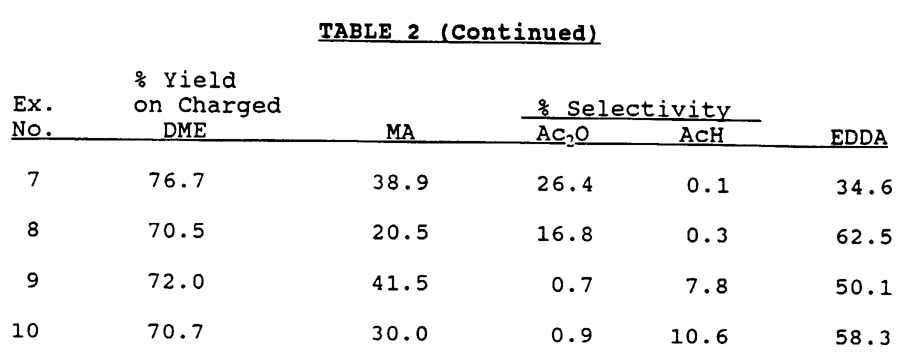

### EXAMPLES 11-14

Examples 11 - 13, set forth in Table 3, illustrate the effect of a hydrogen-rich atmosphere (75 mol%) on organic product distribution obtained by operating the process of the present invention. In particular, use of more than 75 mol% H₂ causes greater amounts of EtOAc being produced along with EtI. The data demonstrate that the present process is preferably run using a 1:1 to 4:1 molar ratio of carbon monoxide to hydrogen, meaning that the hydrocarbonylation should be run rich in carbon monoxide over hydrogen.

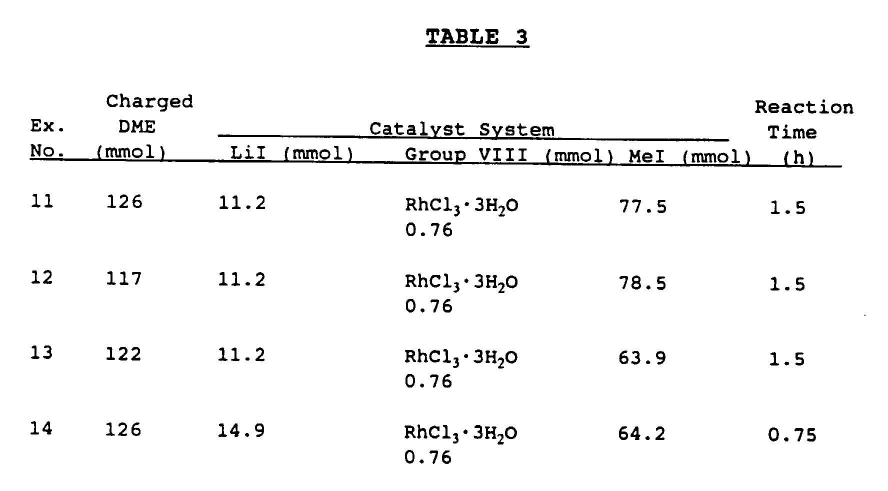

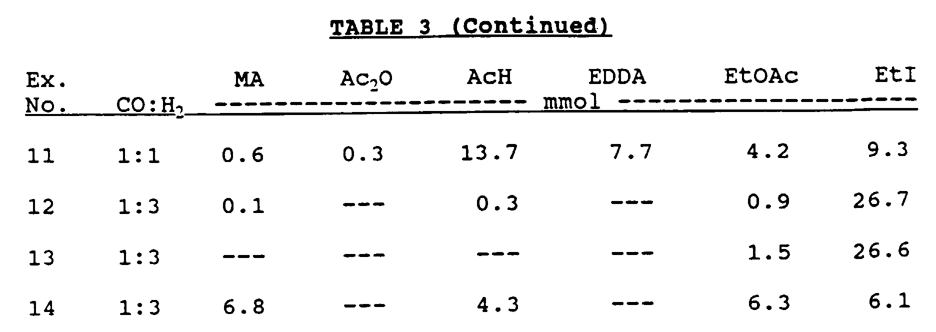

### EXAMPLES 15-16

Examples 15 - 16 set forth in Table 4 provide support to the inverse relationship between EDDA selectivity and mol % LiI/(mol LiI + mol MeI) as noted previously.

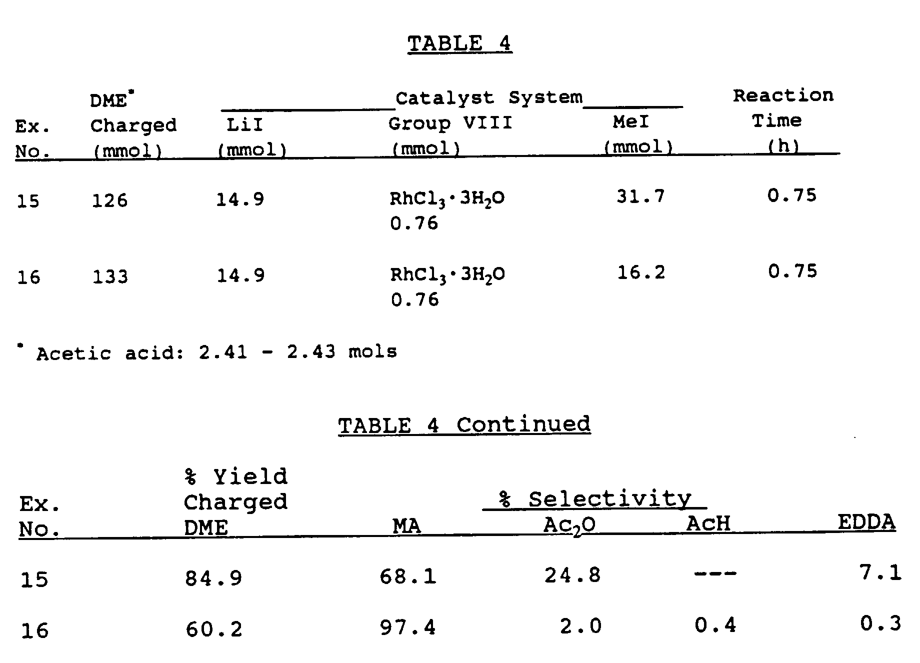

### EXAMPLES 17-26

Examples 17 - 26, set forth in Table 5 use a CO/H₂ mix of 1:1. These Examples illustrate the effect of stirring rate (Examples 20 and 21) and total hydrocarbonylation reaction pressure (Examples 23, 24 and 25) on the percent yield based on charged DME. Under comparable reaction conditions, the percentage yield based on charged DME increased with stirring rate and total reaction pressure. The data also demonstrate that longer reaction times favor EDDA formation under comparable reaction conditions (Examples 25 and 26). Example 22 indicates that no EDDA was detected in the absence of Group VIII metal in the catalyst system. Therefore, the process of the present invention is preferably conducted at pressures of at least 400 psig (2.75 MPa) at a stirring rate in the range of 800 to 2000 rpm.

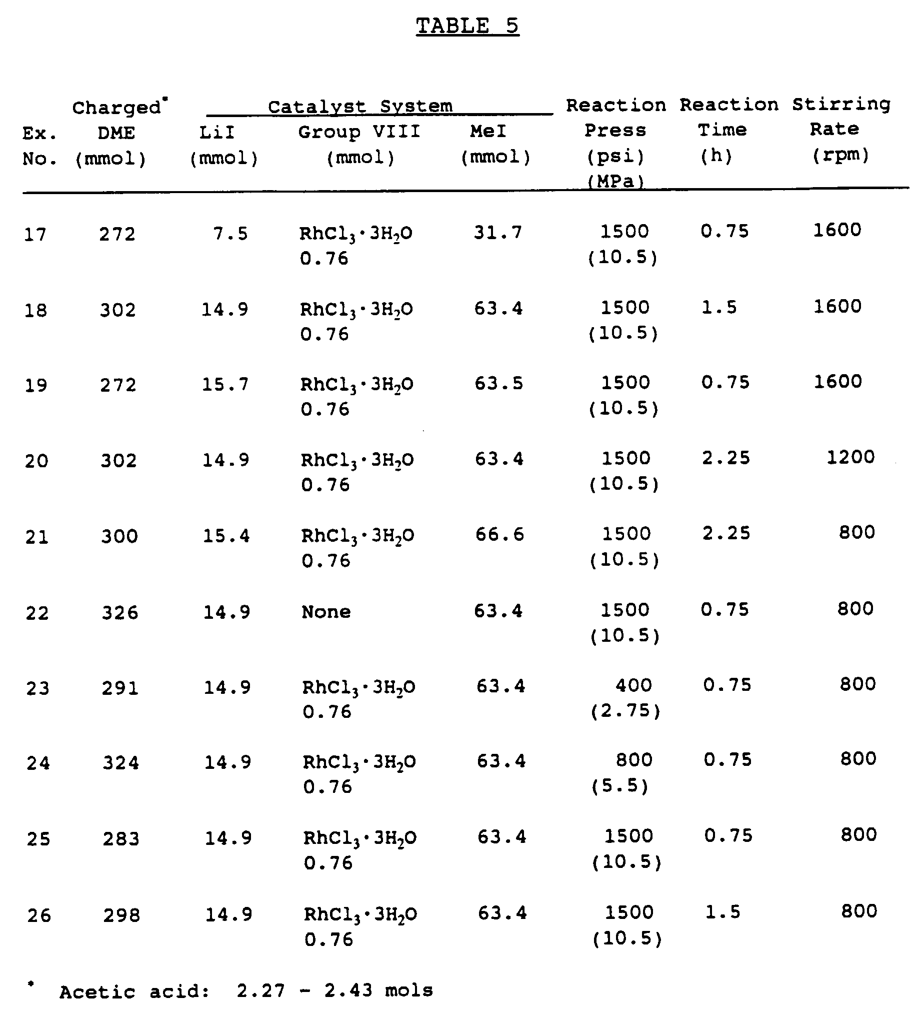

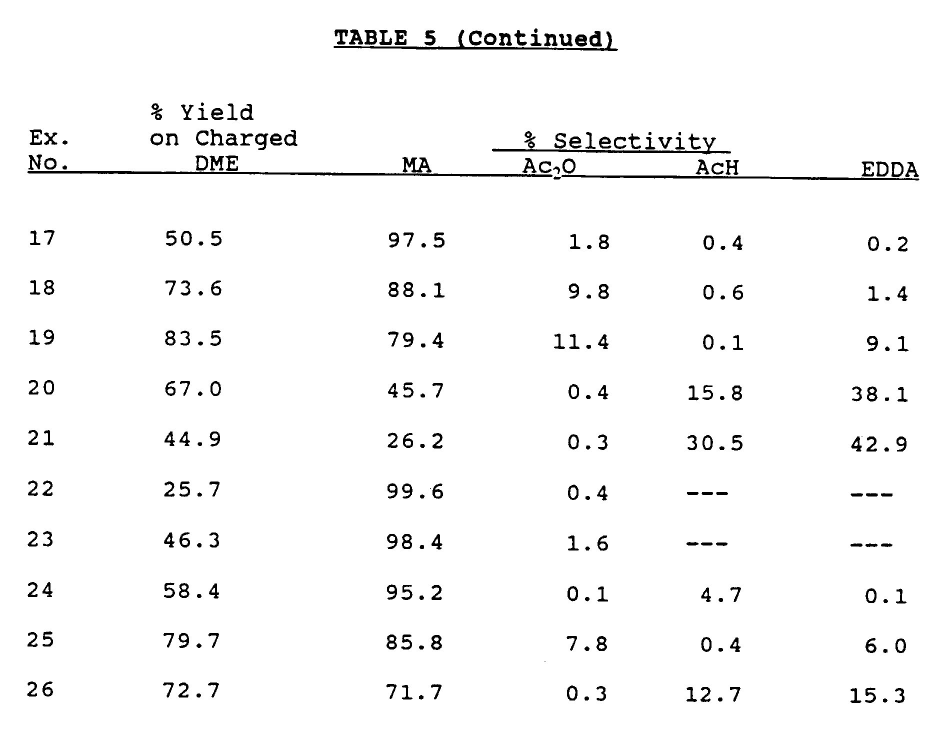

### EXAMPLES 27 - 30

Examples 27 through 30, set forth in Table 6 use a CO/H₂ mix of 1:1 and illustrate the effect of reaction temperature on selectivity to EDDA at approximately the same % yield based on charged DME at a stirring rate of 800 rpm (Examples 27 and 28). A doubling in the amount of LiI was found to decrease the percentage yield by about 30% based on the charged DME (Example 28 and 30). Example 29 demonstrates the activity of methyl iodide in the absence of lithium iodide.

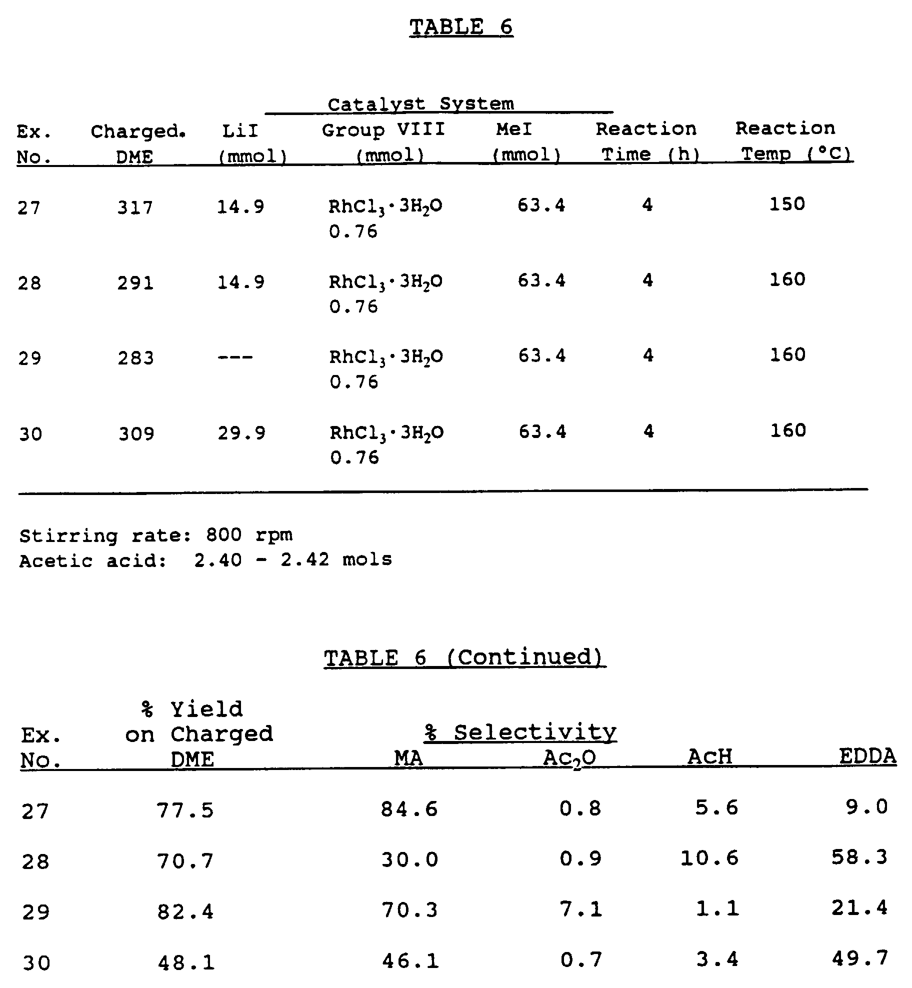

### EXAMPLES 31-32

Examples 31 - 32 set forth in Table 7 below illustrate the effect of introducing lithium acetate into the catalyst system and the resulting increase in conversion of DME to EDDA. Only 80.9% of the DME was converted to organic products in the absence of lithium iodide, while use of lithium acetate resulted in 96.3% conversion to products.

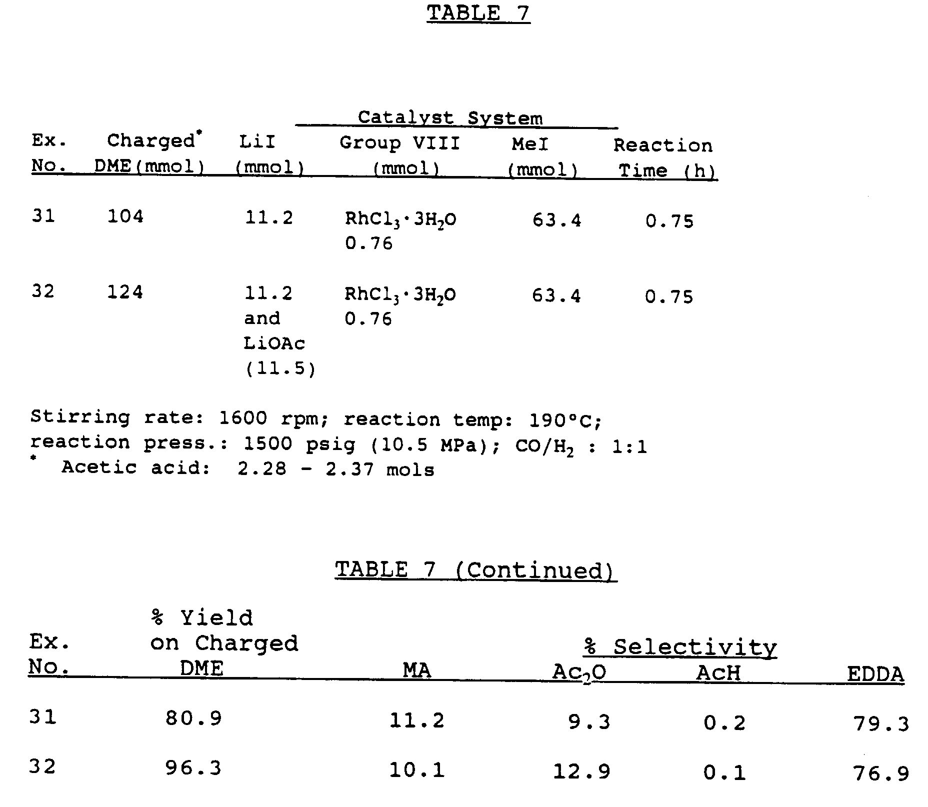

## Claims

1. A process for producing ethylidene diacetate which comprises reacting dimethyl ether, hydrogen, carbon monoxide and acetic acid in the presence of a catalyst system consisting essentially of a Group VIII metal, methyl iodide, lithium iodide and, optionally, lithium acetate to form ethylidene diacetate.

2. A process according to Claim 1, wherein the reaction temperature is from 20°C to 220°C.

3. A process according to Claim 2, wherein the reaction temperature is from 120°C to 195°C.

4. A process according to any one of the preceding claims, wherein the reaction pressure is from 0.8 MPa (100 psig) to 21 MPa (3000 psig).

5. A process according to Claim 4, wherein the reaction pressure is from 2.75 MPa (400 psig) to 14.5 MPa (2100 psig).

6. A process according to any one of the preceding claims, wherein the reaction pressure is greater than 2.75 MPa (400 psi), the reaction time is greater than 0.5 hour and the reactants are stirred at a rate of from 800 to 2000 rpm.

7. A process according to any one of the preceding claims, wherein the catalyst system comprises lithium acetate.

8. A process according to any one of the preceding claims, wherein the Group VIII metal is rhodium.

9. A process according to Claim 8, wherein the source of the Group VIII metal is rhodium (III) chloride trihydrate.

10. A process according to any one of the preceding claims, wherein the molar ratio of carbon monoxide to hydrogen is 1:1 to 4:1.

11. A catalyst system for catalysing the production of ethylidene diacetate by reacting dimethyl ether, hydrogen, carbon monoxide and acetic acid, said catalyst system consisting essentially of a Group VIII metal, methyl iodide, lithium iodide and, optionally, lithium acetate

12. A catalyst system according to Claim 11, wherein the catalyst system comprises lithium acetate.

13. A catalyst system according to Claim 11 or Claim 12, wherein the Group VIII metal is rhodium.

14. A catalyst system according to Claim 13, wherein the source of the Group VIII metal is rhodium (III) chloride trihydrate.

## Patentansprüche

1. Verfahren zur Herstellung von Ethylidendiacetat, umfassend die Umsetzung von Dimethylether, Wasserstoff, Kohlenmonoxid und Essigsäure in Gegenwart eines Katalysatorsystems, das im wesentlichen aus einem Metall der Gruppe VIII, Methyliodid, Lithiumiodid und wahlweise Lithiumacetat besteht, um Ethylidendiacetat zu bilden.

2. Verfahren nach Anspruch 1, worin die Reaktionstemperatur 20°C bis 220°C beträgt.

3. Verfahren nach Anspruch 2, worin die Reaktionstemperatur 120°C bis 195°C beträgt.

4. Verfahren nach einem der vorhergehenden Ansprüche, worin der Reaktionsdruck 0,8 MPa (100 psig) bis 21 MPa (3.000 psig) beträgt.

5. Verfahren nach Anspruch 4, worin der Reaktionsdruck 2,75 MPa (400 psig) bis 14,5 MPa (2.100 psig) beträgt.

6. Verfahren nach einem der vorhergehenden Ansprüche, worin der Reaktionsdruck größer als 2,75 MPa (400 psi) ist, die Reaktionszeit mehr als 0,5 Stunden beträgt und die Reaktionsteilnehmer mit einer Geschwindigkeit von 800 bis 2.000 UpM gerührt werden.

7. Verfahren nach einem der vorhergehenden Ansprüche, worin das Katalysatorsystem Lithiumacetat umfaßt.

8. Verfahren nach einem der vorhergehenden Ansprüche, worin das Metall der Gruppe VIII Rhodium ist.

9. Verfahren nach Anspruch 8, worin die Quelle für das Metall der Gruppe VIII Rhodium(III)chlorid-trihydrat ist.

10. Verfahren nach einem der vorhergehenden Ansprüche, worin das Molverhältnis von Kohlenmonoxid zu Wasserstoff 1:1 bis 4:1 beträgt.

11. Katalysatorsystem zum Katalysieren der Ethylidendiacetatherstellung durch Umsetzung von Dimethylether, Wasserstoff, Kohlenmonoxid und Essigsäure, wobei das Katalysatorsystem im wesentlichen aus einem Metall der Gruppe VIII, Methyliodid, Lithiumiodid und wahlweise Lithiumacetat besteht.

12. Katalysatorsystem nach Anspruch 11, worin das Katalysatorsystem Lithiumacetat umfaßt.

13. Katalysatorsystem nach Anspruch 11 oder Anspruch 12, worin das Metall der Gruppe VIII Rhodium ist.

14. Katalysatorsystem nach Anspruch 13, worin die Quelle für das Metall der Gruppe VIII Rhodium(III)chlorid-trihydrat ist.

## Revendications

1. Procédé pour la production de diacétate d'éthylidène qui comprend la mise en réaction de diméthyléther, d'hydrogène, de monoxyde de carbone et d'acide acétique en présence d'un système catalyseur consistant essentiellement en un métal du Groupe VIII, iodure de méthyle, iodure de lithium et facultativement acétate de lithium pour former du diacétate d'éthylidène.

2. Procédé selon la revendication 1, dans lequel la température de réaction est de 20°C à 220°C.

3. Procédé selon la revendication 2, dans lequel la température de réaction est de 120°C à 195°C.

4. Procédé selon l'une quelconque des revendications précédentes, dans lequel la pression de réaction est de 0,8 MPa (100 psig) à 21 MPa (3000 psig).

5. Procédé selon la revendication 4, dans lequel la pression réactionnelle est de 2,75 MPa (400 psig) à 14,5 MPa (2100 psig).

6. Procédé selon l'une quelconque des revendications précédentes, dans lequel la pression de réaction est supérieure à 2,75 MPa (400 psig), le temps de réaction est supérieur à 0,5 heure et les réactifs sont agités à une vitesse de 800 à 2000 tours/minute.

7. Procédé selon l'une quelconque des revendications précédentes, dans lequel le système catalyseur comprend de l'acétate de lithium.

8. Procédé selon l'une quelconque des revendications précédentes, dans lequel le métal de Groupe VIII est le rhodium.

9. Procédé selon la revendication 8, dans lequel la source de métal de Groupe VIII est le trihydrate de chlorure de rhodium (III).

10. Procédé selon l'une quelconque des revendications précédentes, dans lequel le rapport molaire entre le monoxyde de carbone et l'hydrogène est de 1:1 à 4:1.

11. Système catalyseur destiné à catalyser la production de diacétate d'éthylidène par mise en réaction de diméthyléther, d'hydrogène, de monoxyde de carbone et d'acide acétique, le système catalyseur consistant essentiellement en un métal de Groupe VIII, en un iodure de méthyle, un iodure de lithium et facultativement un acétate de lithium.

12. Système catalyseur selon la revendication 11, dans lequel le système catalyseur comprend de l'acétate de lithium.

13. Système catalyseur selon la revendication 11 ou la revendication 12, dans lequel le métal de Groupe VIII est le rhodium.

14. Système catalyseur selon la revendication 13, dans lequel la source de métal de Groupe VIII est le trihydrate de chlorure de rhodium (III).
